# EUROPÄISCHE PATENTSCHRIFT

(11) **EP 4 126 518 B1**
(45) Veröffentlichungstag und Bekanntmachung des Hinweises auf die Patenterteilung: **24.06.2026**
(21) Anmeldenummer: 21713915.3
(22) Anmeldetag: 18.03.2021
(51) Int. Cl.: B29D 11/00, B29D 11/02, B29C 35/08, B29C 64/124, A61F 2/16, B29C 70/68

(54) **VERFAHREN ZUM HERSTELLEN EINER INTRAOKULARLINSE UND HERSTELLUNGSVORRICHTUNG**
METHOD FOR PRODUCING AN INTRAOCULAR LENS, AND PRODUCTION DEVICE
PROCÉDÉ DE PRODUCTION D'UNE LENTILLE INTRAOCULAIRE ET DISPOSITIF DE PRODUCTION

(30) Priorität: 26.03.2020 DE 102020108375
(43) Veröffentlichungstag der Anmeldung: 08.02.2023
(73) Patentinhaber: Carl Zeiss Meditec AG, 07745 Jena (DE)
(72) Erfinder: THALLER, Michael, 10585 Berlin (DE)
(74) Vertreter: PATERIS Patentanwälte PartmbB
(86) Internationale Anmeldenummer: PCT/EP2021/056896
(87) Internationale Veröffentlichungsnummer: WO 2021/191034

(56) Entgegenhaltungen:
- WO-A1-2019/043529
- WO-A1-2019/220307
- US-A1- 2020 039 118
- US-B2- 10 111 746
- LOTERIE DAMIEN ET AL: "High-resolution tomographic volumetric additivemanufacturing", NATURE COMMUNICATIONS, vol. 11, no. 1, 12 February 2020 (2020-02-12), GB, pages 852 - 852, XP055815318, ISSN: 2041-1723, Retrieved from the Internet <URL:https://www.nature.com/articles/s41467-020-14630-4.pdf> [retrieved on 20210617], DOI: 10.1038/s41467-020-14630-4

## Beschreibung

Die Erfindung betrifft ein Verfahren zum Herstellen einer Intraokularlinse und eine Herstellungsvorrichtung zum Herstellen der Intraokularlinse.

Intraokularlinsen werden herkömmlich durch Drehen hergestellt. Dazu wird zuerst durch Polymerisation das Material der Intraokularlinse hergestellt und anschließend werden aus dem Material Rohlinge geschnitten. Die Rohlinge werden dann mittels eines Wachses an einer Drehmaschine befestigt und ein computergesteuerter Roboterarm, der mit einer Diamantspitze bestückt ist, spant aus dem in der Drehmaschine rotierenden Rohling die Intraokularlinse. Dabei handelt es sich nachteilig um ein aufwändiges und damit kostenintensives Verfahren. Zudem ist nachteilig, dass bei dem Drehen mit der Diamantspitze Rillen auf der Oberfläche der Intraokularlinse entstehen, wobei die Rillen die optische Qualität der Intraokularlinse beeinträchtigen.

US 10 111 746 B2 beschreibt ein Verfahren zum Herstellen einer Intraokularlinse. US 2020/039118 A1 offenbart eine statische flüssige Grenzflächenherstellung von Linsen. WO 2019/220307 A1 offenbart ein Gerät zum Ernten von Energie durch eine relative Bewegung von Körperteilen. LOTERIE DAMIEN ET AL: "Highresolution tomographic volumetric additive manufacturing", NATURE COMMUNICATIONS, Bd. 11, Nr. 1, 12. Februar 2020 (2020-02-12), Seiten 852-852, XP055815318, GB ISSN: 2041-1723, DOI: 10.1038/s41467-020-14630-4 offenbart eine additives Herstellungsverfahren. WO 2019/043529 A1 offenbart ein Verfahren und eine Vorrichtung für dreidimensionales Fertigstellen mittels tomographischer Projektionen.

Aufgabe der Erfindung ist daher, ein Verfahren zum Herstellen einer Intraokularlinse und eine Herstellungsvorrichtung zum Herstellen der Intraokularlinse zu schaffen, mit denen die vorgenannten Probleme überkommen werden können.

Das erfindungsgemäße Verfahren zum Herstellen einer Intraokularlinse weist die Schritte auf: - Bereitstellen eines für elektromagnetische Strahlung transparenten Behälters, in dem eine Flüssigkeit angeordnet ist, die mit der elektromagnetischen Strahlung aushärtbar ist; - Bestrahlen der Flüssigkeit mit einem Satz an Bildern , die von der elektromagnetischen Strahlung gebildet werden und die jeweils eine Intraokularlinse zeigen, wobei jedes der Bilder des Satzes unter einem anderen Einstrahlwinkel bezüglich einer durch die Flüssigkeit verlaufenden Bezugsebene in die Flüssigkeit eingestrahlt wird, wodurch die Flüssigkeit ausgehärtet wird und die ausgehärtete Flüssigkeit die Intraokularlinse bildet. Dabei handelt es sich um ein vorteilhaft einfaches Verfahren zum Herstellen der Intraokularlinse. Zudem entstehen durch das Verfahren auf der Oberfläche der Intraokularlinse keine Rillen.

Die Bilder des Satzes können beispielsweise aus einem dreidimensionalen Datensatz, der die Form der Intraokularlinse beinhaltet, errechnet werden. Dabei handelt es sich um einen umgekehrten Prozess, wie er bei einer tomographischen Bildgebung eingesetzt wird. Die tomographische Bildgebung kommt beispielsweise bei einer Computertomographie zum Einsatz. Die tomographische Bildgebung kann beispielsweise von einer Radon-Transformation Gebrauch machen.

Die Flüssigkeit kann beispielsweise Monomere eines Polyacrylats oder Monomere eines Silikons aufweisen. Zudem kann die Flüssigkeit einen Photoinitiator aufweisen, wie beispielsweise Campherchinon.

Es ist bevorzugt, dass die Intraokularlinse eine akkommodierende Intraokularlinse ist. Die akkommodierende Intraokularlinse zeichnet sich dadurch aus, dass sie einen Optikkörper aufweist, der besonders weich ist und somit die Fähigkeit hat, sich zu verformen und somit seinen Brennpunkt zu verschieben. Wird die Intraokularlinse herkömmlich mittels Drehen hergestellt, ist eine gewisse Mindeststeifigkeit des Materials der Intraokularlinse Voraussetzung. Mit dieser Mindeststeifigkeit ist es jedoch nicht möglich, die Intraokularlinse so weich auszuführen, dass damit die akkommodierende Intraokularlinse geschaffen werden kann. Bei dem erfindungsgemäßen Verfahren fällt jedoch das Erfordernis der Mindeststeifigkeit weg, so dass die akkommodierende Intraokularlinse hergestellt werden kann. Beispielsweise durch eine Wahl der Monomere, eines Massenverhältnisses der Monomere, eines Katalysators, die Temperatur der Flüssigkeit und/oder von Reaktionszeiten können die Eigenschaften der Intraokularlinse und insbesondere die Steifigkeit der Intraokularlinse eingestellt werden.

Es ist bevorzugt, dass die Flüssigkeit Sauerstoff und/oder einen Radikalfänger aufweist, wobei der Sauerstoff und/oder der Radikalfänger in der Flüssigkeit gelöst ist. Unter dem Radikalfänger werden organische oder anorganische Substanzen verstanden, deren chemische Reaktion mit reaktiven Radikalen zu stabileren Verbindungen führt, wobei der Sauerstoff hier nicht zu den Radikalfängern gezählt wird. Das Aushärten der Flüssigkeit kann von einer radikalischen Polymerisation verursacht werden. Dazu kann die Flüssigkeit den Photoinitiator aufweisen und die Monomere können eingerichtet sein, eine radikalische Polymerisation einzugehen. Bei dem Bestrahlen der Flüssigkeit zerfällt der Photoinitiator und bildet dadurch Radikale. Die Radikale reagieren mit dem Monomer und durch Kettenwachstum wird aus dem Monomer das Polymer gebildet. Der Sauerstoff und/oder der Radikalfänger sorgen dafür, dass das Kettenwachstum abgebrochen oder erst gar nicht gestartet wird und somit das Aushärten der Flüssigkeit unterbleibt. Dies wird beispielhaft durch die untenstehenden Reaktionsgleichungen illustriert, wobei A das Monomer bezeichnet und I-I den Photoinitiator bezeichnet:
a) 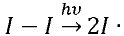
*b)*

   *I ·* +*A* → *I + A·*
*c)*

   *A· +A* → *A - A **·***
d)

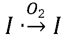

In Reaktionsgleichung a) ist der Zerfall des Initiators dargestellt. In Reaktionsgleichung b) wird ein Radikal des Monomers erzeugt und in Reaktionsgleichung c) ist das Kettenwachstum dargestellt. In Reaktionsgleichung d) ist ein Quenchen der Radikale des zerfallenen Photoinitiators dargestellt. Erst wenn der Sauerstoff und/oder der Radikalfänger verbraucht sind, kann die Flüssigkeit ausgehärtet werden. Der Sauerstoff und/oder der Radikalfänger werden zuerst in den Bereichen in der Flüssigkeit verbraucht, die stärker als die anderen Bereiche der Flüssigkeit bestrahlt werden. Dadurch kann erreicht werden, dass nur die Bereiche der Flüssigkeit, die die Intraokularlinse bilden sollen, ausgehärtet werden.

Die Flüssigkeit kann in einem ersten Beispiel folgende Zusammensetzung haben: 96,97 Gew.-% Polydimethylsiloxan mit endständigen Vinylgruppen, 3,00 Gew.-% (Mercaptopropyl)Methylsiloxan-dimethylsiloxan und 0,03 Gew.-% Campherchinon.

In einem zweiten Beispiel kann die Flüssigkeit folgende Zusammensetzung aufweisen: 99,98 Gew.-% aliphatische Urethandiacrylsäure und 0,02 Gew.-% Campherchinon.

In dem ersten Beispiel und in dem zweiten Beispiel wurde durch die Flüssigkeit 2 h gasförmiger Sauerstoff geleitet, um die Flüssigkeit mit Sauerstoff zu sättigen. Mit dieser Flüssigkeit konnte eine Intraokularlinse hergestellt werden, die derart weich ist, dass sie als akkommodiere Intraokularlinse geeignet ist.

Es ist bevorzugt, dass die Bilder gleichzeitig in die Flüssigkeit eingestrahlt werden. Dadurch kann die Intraokularlinse besonders schnell hergestellt werden.

Alternativ ist bevorzugt, dass die Bilder nacheinander und während der Behälter rotiert wird in die Flüssigkeit eingestrahlt werden, wobei die Bezugsebene zusammen mit dem Behälter rotiert. Hier ist vorteilhaft nur ein einzelner Projektor erforderlich, um die Flüssigkeit mit dem Satz der Bilder zu bestrahlen. Es ist besonders bevorzugt, dass der Satz wiederholt eingestrahlt wird und gleiche Bilder der wiederholt eingestrahlten Sätze bei dem gleichen Einstrahlwinkel in die Flüssigkeit eingestrahlt werden.

Es ist erfindungsgemäß, dass in der Flüssigkeit ein Aktuator, ein Solarmodul und/oder ein Sensor angeordnet ist, und dass die Intraokularlinse um den Aktuator, das Solarmodul und/oder den Sensor herum gebildet wird. Dadurch können zusätzlich zu der abbildenden Funktionalität der Intraokularlinse weitere Funktionalitäten in die Intraokularlinse eingearbeitet werden.

Das Verfahren weist bevorzugt den Schritt auf: - Nacharbeiten der Intraokularlinse, nachdem sie gebildet wurde. Besonders bevorzugt wird mittels Drehen und/oder Laserabtrag nachgearbeitet. Dadurch ist es möglich, die abbildenden Eigenschaften der Intraokularlinse zu verbessern.

Es ist bevorzugt, dass die Intraokularlinse einen Optikkörper und mindestens zwei Haptiken aufweist, wobei jede der Haptiken einen ersten Haptikarm und einen zweiten Haptikarm aufweist, die an der gleichen Stelle an dem Optikkörper angebracht sind und in einer Ebene, in der die optische Achse des Optikkörpers angeordnet ist, einen Winkel einschließen, der größer als Null ist. Dabei handelt es sich um Haptiken, die herkömmlich mittels Drehen nicht hergestellt werden können.

Die Intraokularlinse weist bevorzugt einen Hohlraum auf. Der Hohlraum kann herkömmlich mittels Drehen nicht hergestellt werden.

Die erfindungsgemäße Herstellungsvorrichtung für eine Intraokularlinse weist einen für elektromagnetische Strahlung transparenten Behälter und eine Projektionsvorrichtung auf, die eingerichtet ist, einen Innenraum des Behälters mit einem Satz an Bildern zu bestrahlen, die von der elektromagnetischen Strahlung gebildet werden und die jeweils die Intraokularlinse zeigen, und jedes der Bilder des Satzes unter einem anderen Einstrahlwinkel bezüglich einer durch den Innenraum verlaufenden Bezugsebene in den Innenraum einzustrahlen.

Die Herstellungsvorrichtung weist erfindungsgemäß die Flüssigkeit auf, die in dem Innenraum angeordnet ist und mit der elektromagnetischen Strahlung aushärtbar ist.

Es ist erfindungsgemäß, dass in der Flüssigkeit ein Aktuator, ein Solarmodul und/oder ein Sensor angeordnet ist, und dass die Herstellungsvorrichtung eingerichtet ist, die Intraokularlinse um den Aktuator, das Solarmodul und/oder den Sensor herum zu bilden.

Die Herstellungsvorrichtung weist bevorzugt eine Speichereinheit auf, in der die Bilder gespeichert sind. Dabei ist denkbar, dass die Speichereinheit ein Teil der Projektionsvorrichtung ist.

Es ist bevorzugt, dass die Projektionsvorrichtung für jedes der Bilder des Satzes jeweils einen Projektor aufweist, der eingerichtet ist, den Innenraum des Behälters mit dem zu dem Projektor zugehörigen Bild zu bestrahlen. Dabei ist denkbar, dass jeder der Projektoren einen Teil der Speichereinheit aufweist.

Alternativ ist es bevorzugt, dass die Herstellungsvorrichtung eine Drehvorrichtung aufweist, die eingerichtet ist, den Behälter zusammen mit der Bezugsebene in eine Rotation zu versetzen, deren Symmetrieachse durch den Behälter verläuft, und die Projektionsvorrichtung einen Projektor aufweist, der eingerichtet ist, die Bilder des Satzes nacheinander in den Behälter einzustrahlen. Dabei ist besonders bevorzugt, dass die Herstellungsvorrichtung eine Mehrzahl der Behälter und für jeden der Behälter eine Drehvorrichtung aufweist, die eingerichtet ist, den zugehörigen Behälter in die Rotation zu versetzen, deren Symmetrieachse durch den zugehörigen Behälter verläuft, und die Projektionsvorrichtung eingerichtet ist, in jeden Innenraum von jedem der Behälter einen jeweiligen Satz an Bildern, die von der elektromagnetischen Strahlung gebildet werden und die jeweils eine Intraokularlinse zeigen, einzustrahlen, wobei die Projektionsvorrichtung eingerichtet ist, die Behälter gleichzeitig zu bestrahlen. Dadurch ist es möglich, eine Mehrzahl der Intraokularlinsen gleichzeitig herzustellen. Dabei ist es denkbar, dass die Projektionsvorrichtung für jeden der Behälter jeweils einen Projektor aufweist, der eingerichtet ist, den zugehörigen Behälter zu bestrahlen. Dadurch ist es möglich, die Mehrzahl der Intraokularlinsen mit einer unterschiedlichen Form herzustellen. Alternativ ist es denkbar, dass die Projektionsvorrichtung einen einzelnen Projektor aufweist, dessen Strahlengang mittels mindestens einen Strahlenteiler in Teilstrahlengänge aufgeteilt wird, die eingerichtet sind, jeweils einen der Behälter zu bestrahlen. Dadurch ist vorteilhaft nur ein Projektor notwendig, um zeitgleich eine Mehrzahl der Intraokularlinse herzustellen.

Im Folgenden wird anhand der beigefügten schematischen Zeichnungen die Erfindung näher erläutert.
Figur 1 zeigt einen horizontalen Schnitt durch eine erste Ausführungsform einer erfindungsgemäßen Herstellungsvorrichtung.
Figur 2 zeigt einen vertikalen Schnitt durch die erste Ausführungsform.
Figur 3 zeigt einen horizontalen Schnitt durch eine zweite Ausführungsform der erfindungsgemäßen Herstellungsvorrichtung.
Figur 4 veranschaulicht die Herstellung einer Intraokularlinse.
Figur 5 zeigt eine erste Ausführungsform der Intraokularlinse.
Figur 6 zeigt eine zweite Ausführungsform der Intraokularlinse.
Figur 7 zeigt eine dritte Ausführungsform der Intraokularlinse.
Figur 8 zeigt eine vierte Ausführungsform der Intraokularlinse.
Figur 9 zeigt eine fünfte Ausführungsform der Intraokularlinse.

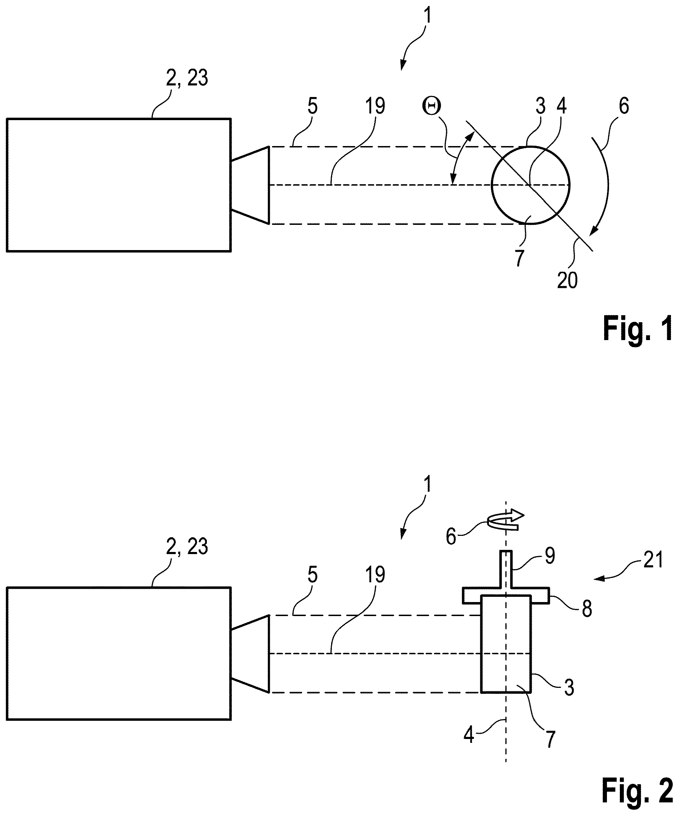

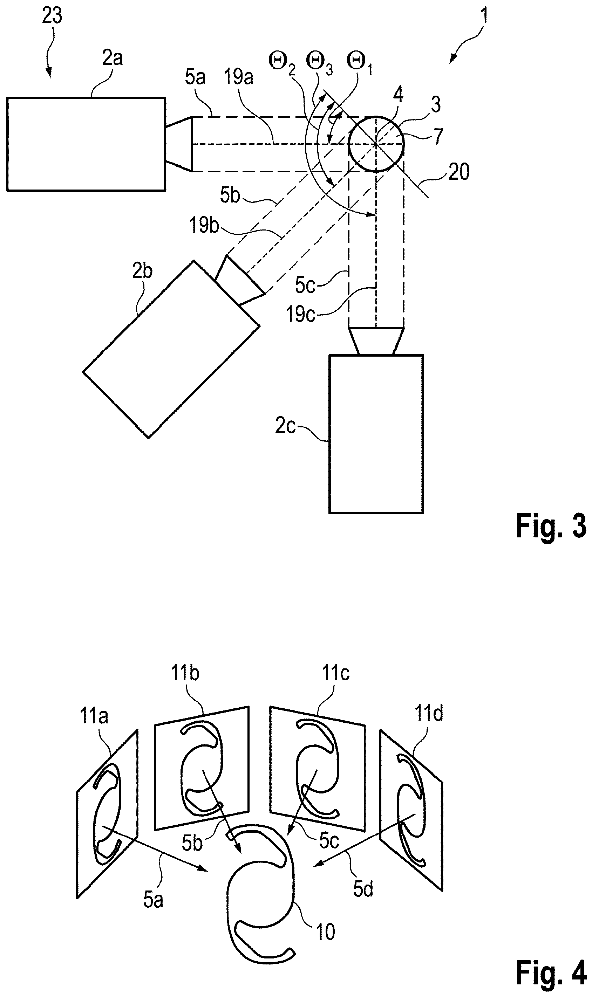

Wie es aus Figuren 1 bis 3 ersichtlich ist, weist eine erfindungsgemäße Herstellungsvorrichtung 1 für eine Intraokularlinse 10 einen für elektromagnetische Strahlung transparenten Behälter 3 und eine Projektionsvorrichtung 23 auf, die eingerichtet ist, einen Innenraum des Behälters 3 mit einem Satz an Bildern 11 zu bestrahlen, die von der elektromagnetischen Strahlung gebildet werden und die jeweils die Intraokularlinse 10 zeigen. Zudem ist die Projektionsvorrichtung 23 eingerichtet, jedes der Bilder 11 des Satzes unter einem anderen Einstrahlwinkel Θ bezüglich einer durch den Innenraum verlaufenden Bezugsebene 20 in den Innenraum einzustrahlen. Figuren 1 und 3 zeigen, dass der Einstrahlwinkel Θ definiert sein kann als der Winkel zwischen einer optischen Achse 19 eines Strahlengangs 5 der auf den Behälter 3 auftreffenden elektromagnetischen Strahlung. Aus Figuren 1 bis 3 ist ersichtlich, dass eine Wand des Behälters 3 die Form eines Mantels eines Zylinders haben kann, wodurch der Innenraum des Behälters 3 die Form eines Zylinders hat. Damit hat der Innenraum des Behälters 3 eine Symmetrieachse 4, die die Achse des Zylinders ist. Es ist denkbar, dass die Symmetrieachse 4 in der Bezugsebene 20 liegt, wie es auch in Figuren 1 und 3 dargestellt ist.

Die Bildebene des Strahlengangs 5 kann so angeordnet sein, dass die Bildebene in dem Innenraum des Behälters 3 liegt, insbesondere kann die Bildebene so angeordnet sein, dass die Symmetrieachse 4 in der Bildebene liegt. Es ist zudem denkbar, dass eine Tiefenschärfe, die sich ausgehend von der Bildebene strahlaufwärts und strahlabwärts der Bildebene erstreckt, länger als die Erstreckung der optischen Achse 19 in dem Innenraum ist. Außerdem ist denkbar, dass der Strahlengang 5 zumindest einen kreisförmigen Querschnitt des Innenraums vollständig ausleuchtet. Bei der elektromagnetischen Strahlung kann es sich beispielhaft um sichtbares Licht und/oder Ultraviolettstrahlung handeln.

Die Herstellungsvorrichtung 1 kann eine Speichereinheit aufweisen, in der die Bilder 11 gespeichert sind. Die Speichereinheit kann beispielsweise ein Teil der Projektionsvorrichtung 23 sein.

Figuren 1 bis 3 zeigen, dass die Herstellungsvorrichtung 1 eine Flüssigkeit 7 aufweisen kann, die in dem Innenraum angeordnet ist und mit der elektromagnetischen Strahlung aushärtbar ist. Die Flüssigkeit 7 kann beispielsweise Monomere eines Polyacrylats oder Monomere eines Silikons aufweisen. Zudem kann die Flüssigkeit einen Photoinitiator aufweisen, wie beispielsweise Campherchinon. Die Flüssigkeit 7 kann Sauerstoff und/oder einen Radikalfänger aufweisen, wobei der Sauerstoff und/oder der Radikalfänger in der Flüssigkeit gelöst ist. Unter dem Radikalfänger werden organische oder anorganische Substanzen verstanden, deren chemische Reaktion mit reaktiven Radikalen zu stabileren Verbindungen führt, wobei der Sauerstoff hier nicht zu den Radikalfängern gezählt wird. Das Aushärten der Flüssigkeit 7 kann von einer radikalischen Polymerisation verursacht werden. Dazu kann die Flüssigkeit 7 den Photoinitiator aufweisen und die Monomere können eingerichtet sein, eine radikalische Polymerisation einzugehen.

Eine erste Ausführungsform der Herstellungsvorrichtung 1 gemäß Figuren 1 und 2 weist eine Drehvorrichtung 21 auf, die eingerichtet ist, den Behälter 3 zusammen mit der Bezugsebene 20 in eine Rotation zu versetzen, deren Symmetrieachse 4 durch den Behälter 3 verläuft, und die Projektionsvorrichtung 23 weist einen Projektor 2 auf, der eingerichtet ist, die Bilder 11 des Satzes nacheinander in den Behälter 3 einzustrahlen. Die Bezugsebene 20 und der Behälter 3 rotieren dabei mit derselben Winkelgeschwindigkeit. Die Symmetrieachse 4 der Rotation kann mit der Symmetrieachse des Behälters 3 zusammenfallen. Eine beispielhafte Drehrichtung 6 ist in Figuren 1 und 2 eingezeichnet. Die Drehvorrichtung 21 kann einen Halter 8 aufweisen, der den Behälter 3 von oben umgreift. Zudem kann die Drehvorrichtung 21 eine Drehachse 9 aufweisen, die an dem Halter 8 fest angebracht ist und die eingerichtet ist, in einem Betrieb der Drehvorrichtung 21 von einem Motor der Drehvorrichtung in die Rotation versetzt zu werden.

Es ist für die erste Ausführungsform denkbar, dass die Herstellungsvorrichtung 1 eine Mehrzahl der Behälter 3 und für jeden der Behälter 3 eine Drehvorrichtung 21 aufweist, die eingerichtet ist, den zugehörigen Behälter 3 in die Rotation zu versetzen, deren Symmetrieachse 4 durch den zugehörigen Behälter 3 verläuft, und dass die Projektionsvorrichtung 23 eingerichtet ist, in jeden Innenraum von jedem der Behälter 3 einen jeweiligen Satz an Bildern 11, die von der elektromagnetischen Strahlung gebildet werden und die jeweils eine Intraokularlinse 10 zeigen, einzustrahlen, wobei die Projektionsvorrichtung 23 eingerichtet ist, die Behälter 3 gleichzeitig zu bestrahlen. Dabei ist es denkbar, dass die Projektionsvorrichtung 23 für jeden der Behälter 3 jeweils einen Projektor 2 aufweist, der eingerichtet ist, den zugehörigen Behälter 3 zu bestrahlen. Dabei ist denkbar, dass die Projektoren 2 eingerichtet sind, jeweils einen unterschiedlichen Satz der Bilder 11 in den jeweiligen Innenraum einzustrahlen. Alternativ ist es denkbar, dass die Projektionsvorrichtung 23 einen einzelnen Projektor 2 aufweist, dessen Strahlengang 5 mittels mindestens einen Strahlenteiler in Teilstrahlengänge aufgeteilt wird, die eingerichtet sind, jeweils einen der Behälter 3 zu bestrahlen.

Bei einer zweiten Ausführungsform der Herstellungsvorrichtung 1 gemäß Figur 3 weist die Projektionsvorrichtung 23 für jedes der Bilder 11 des Satzes jeweils einen Projektor 2a, 2b, 2c auf, der eingerichtet ist, den Innenraum des Behälters 3 mit dem zu dem Projektor 2 zugehörigen Bild 11 zu bestrahlen. Wie es aus Figur 3 ersichtlich ist, gehört zu jedem der Projektoren 2a, 2b, 2c jeweils ein Strahlengang 5a, 5b, 5c mit jeweils einer optischen Achse 19a, 19b, 19c, wobei jede der optischen Achsen 19a, 19b, 19c jeweils einen anderen Einstrahlwinkel θ₁, θ₂, θ₃ mit der Bezugsebene 20 einschließt.

In Figur 4 ist ein beispielhafter Satz der Bilder 11a bis 11d veranschaulicht, mit dem die Intraokularlinse 10 herstellbar ist, wobei gut erkennbar ist, dass jedes der Bilder 11a bis 11d von einem unterschiedlichen Strahlengang 5a bis 5d gebildet wird und jeder der Strahlengänge 5a bis 5d unter einem anderen Einstrahlwinkel θ bezüglich der Bezugsebene 20 eingestrahlt wird. Die Bilder 11 des Satzes können beispielsweise aus einem dreidimensionalen Datensatz, der die Form der Intraokularlinse 10 beinhaltet, errechnet werden. Dabei handelt es sich um einen umgekehrten Prozess, wie er bei einer tomographischen Bildgebung eingesetzt wird. Die tomographische Bildgebung kommt beispielsweise bei einer Computertomographie zum Einsatz. Die tomographische Bildgebung kann beispielsweise von einer Radon-Transformation Gebrauch machen.

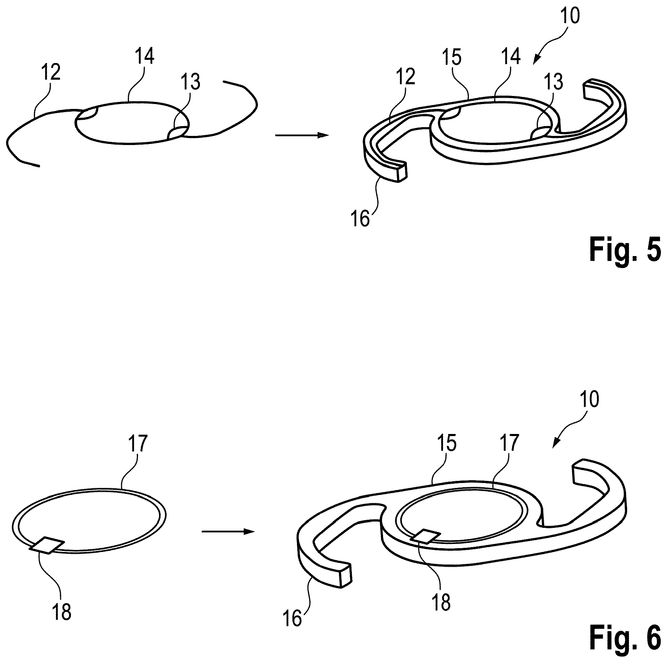

Figur 5 zeigt eine erste Ausführungsform der Intraokularlinse 10, die dadurch hergestellt wurde, dass in der Flüssigkeit 7 ein Aktuator 13 angeordnet wurde und die Intraokularlinse 10 um den Aktuator 13 herum gebildet wurde. Bei der ersten Ausführungsform der Intraokularlinse 10 ist es möglich, eine Haptik 16 der Intraokularlinse 10 zu verbiegen, nachdem die Intraokularlinse 10 in einen Kapselsack eines Auges eingesetzt wurde. Dadurch ist möglich, die Position der Intraokularlinse 10 nachträglich zu verändern, beispielsweise um die Intraokularlinse 10 zu zentrieren oder zu drehen. Dazu weist die Intraokularlinse 10 einen Ring 14 und für jede der Haptiken 16 einen biegbaren Arm 12, der an dem Ring 14 fest angebracht ist, und für jede der Haptiken 16 einen der Aktuatoren 13 auf. Jeder der Aktuatoren 13 ist eingerichtet, den Winkel zwischen dem biegbaren Arm 12 und dem Ring 14 zu verändern. Links in Figur 5 ist die Anordnung aus dem Ring 13, den biegbaren Armen 12 und den Aktuatoren 13 dargestellt, wie sie in die Flüssigkeit 7 einzusetzen ist. Rechts in Figur 5 ist die fertiggestellte Intraokularlinse 10 dargestellt, wobei jede der Haptiken 16 um einen der bewegbaren Arme 12 und ein Optikkörper 15 der Intraokularlinse 10 um den Ring 14 gebildet wurde. Um die Aktuatoren 13 mit Energie zu versorgen, kann die Intraokularlinse 10 zudem ein Solarmodul aufweisen, das in der Flüssigkeit 7 angeordnet wurde und um das die Intraokularlinse 10 herum gebildet wurde sowie das eingerichtet ist, Licht in elektrischen Strom umzuwandeln.

Figur 6 zeigt eine zweite Ausführungsform der Intraokularlinse 10, die dadurch hergestellt wurde, dass in der Flüssigkeit 7 ein Sensor 18 angeordnet wurde und die Intraokularlinse 10 um den Sensor 18 herum gebildet wurde. Die Intraokularlinse 10 kann zudem einen Schaltkreis 17 und/oder ein Solarmodul aufweisen, das eingerichtet ist, Licht in Strom umzuwandeln und den Sensor 18 und/oder den Schaltkreis mit Strom zu versorgen. Links in Figur 6 ist die Anordnung aus dem Sensor 18 und optional dem Schaltkreis 17 und/oder dem Solarmodul dargestellt, wie sie in die Flüssigkeit 7 einzusetzen ist. Rechts in Figur 6 ist die fertiggestellte Intraokularlinse 10 dargestellt.

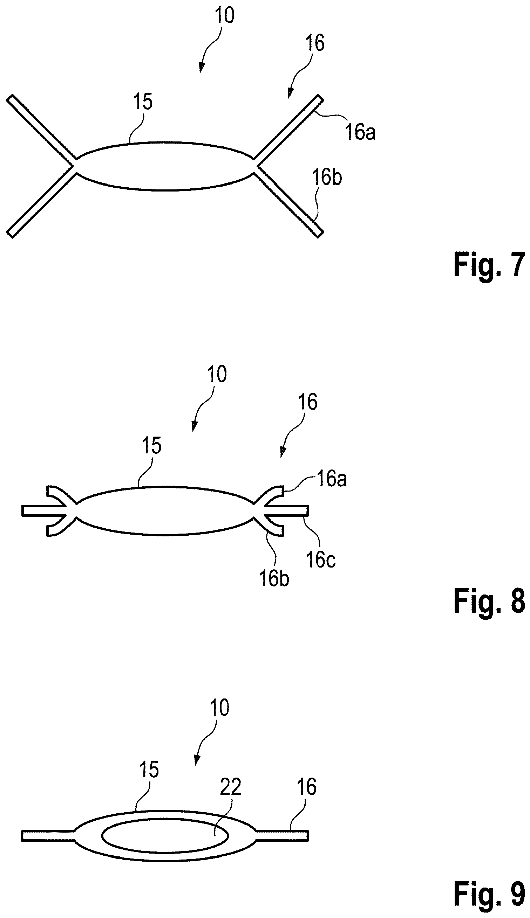

In Figur 7 ist eine dritte Ausführungsform der Intraokularlinse 10 und in Figur 8 ist eine vierte Ausführungsform der Intraokularlinse 10 dargestellt, bei denen die Intraokularlinse 10 einen Optikkörper 15 und mindestens zwei Haptiken 16 aufweist, wobei jede der Haptiken 16 einen ersten Haptikarm 16a und einen zweiten Haptikarm 16b aufweist, die an der gleichen Stelle an dem Optikkörper 15 angebracht sind und in einer Ebene, in der die optische Achse des Optikkörpers 15 angeordnet ist, einen Winkel einschließen, der größer als Null ist. Wie es aus Figur 7 ersichtlich, kann gemäß der dritten Ausführungsform jede der Haptiken 16 aus dem ersten Haptikarm 16a und dem zweiten Haptikarm 16b bestehen. Wie es aus Figur 8 ersichtlich ist, kann gemäß der vierten Ausführungsform jede der Haptiken 16 einen dritten Haptikarm 16c aufweisen, der an der gleichen Stelle wie der erste Haptikarm 16a und der zweite Haptikarm 16b an dem Optikkörper 15 angebracht ist und zwischen dem ersten Haptikarm 16a und dem zweiten Haptikarm 16b angeordnet ist.

In Figur 9 ist eine fünfte Ausführungsform der Intraokularlinse 10 dargestellt, die einen Hohlraum 22 aufweist. Der Hohlraum 22 kann beispielsweise in einem Optikkörper 15 der Intraokularlinse 10 angeordnet sein. Alternativ oder zusätzlich ist denkbar, dass der Hohlraum 22 in einer Haptik 16 der Intraokularlinse angeordnet ist.

### Bezugszeichenliste

1 Herstellungsvorrichtung
2 Projektor
2a Projektor
2b Projektor
2c Projektor
3 Behälter
4 Symmetrieachse
5 Strahlengang
5a Strahlengang
5b Strahlengang
5c Strahlengang
5d Strahlengang
6 Drehrichtung
7 Flüssigkeit
8 Halter
9 Drehachse
10 Intraokularlinse
11 Bild
11a Bild
11b Bild
11c Bild
11d Bild
12 biegbarer Arm
13 Aktuator
14 Ring
15 Optikkörper
16 Haptik
16a erster Haptikarm
16b zweiter Haptikarm
16c dritter Haptikarm
17 Schaltkreis
18 Sensor
19 optische Achse
19a optische Achse
19b optische Achse
19c optische Achse
20 Bezugsebene
21 Drehvorrichtung
22 Hohlraum
23 Projektionsvorrichtung
Θ Einstrahlwinkel
Θ₁ Einstrahlwinkel
Θ₂ Einstrahlwinkel
Θ₃ Einstrahlwinkel

## Patentansprüche

1. Verfahren zum Herstellen einer Intraokularlinse (10), mit den Schritten:
- Bereitstellen eines für elektromagnetische Strahlung transparenten Behälters (3), in dem eine Flüssigkeit angeordnet ist, die mit der elektromagnetischen Strahlung aushärtbar ist;
- Bestrahlen der Flüssigkeit (7) mit einem Satz an Bildern (11), die von der elektromagnetischen Strahlung gebildet werden und die jeweils eine Intraokularlinse (10) zeigen, wobei jedes der Bilder (11) des Satzes unter einem anderen Einstrahlwinkel (Θ) bezüglich einer durch die Flüssigkeit (7) verlaufenden Bezugsebene (20) in die Flüssigkeit eingestrahlt wird, wodurch die Flüssigkeit (7) ausgehärtet wird und die ausgehärtete Flüssigkeit (7) die Intraokularlinse (10) bildet, wobei in der Flüssigkeit (7) ein Aktuator (13), ein Solarmodul und/oder ein Sensor (18) angeordnet ist, und die Intraokularlinse (10) um den Aktuator (13), das Solarmodul und/oder den Sensor (18) herum gebildet wird.

2. Verfahren gemäß Anspruch 1, wobei die Intraokularlinse (10) eine akkommodierende Intraokularlinse (10) ist, die sich dadurch auszeichnet, dass sie einen Optikkörper (15) aufweist, der besonders weich ist und somit die Fähigkeit hat, sich zu verformen und somit seinen Brennpunkt zu verschieben.

3. Verfahren gemäß Anspruch 1 oder 2, wobei die Flüssigkeit (7) Sauerstoff und/oder einen Radikalfänger aufweist, wobei der Sauerstoff und/oder der Radikalfänger in der Flüssigkeit (7) gelöst ist.

4. Verfahren gemäß einem der Ansprüche 1 bis 3, wobei die Bilder (11) gleichzeitig in die Flüssigkeit (7) eingestrahlt werden.

5. Verfahren gemäß einem der Ansprüche 1 bis 3, wobei die Bilder (11) nacheinander und während der Behälter (3) rotiert wird in die Flüssigkeit (7) eingestrahlt werden, wobei die Bezugsebene (20) zusammen mit dem Behälter (3) rotiert.

6. Verfahren gemäß einem der Ansprüche 1 bis 5, mit dem Schritt:
- Nacharbeiten der Intraokularlinse (10), nachdem sie gebildet wurde, insbesondere wird mittels Drehen und/oder Laserabtrag nachgearbeitet.

7. Herstellungsvorrichtung für eine Intraokularlinse (10), mit einem für elektromagnetische Strahlung transparenten Behälter (3) und einer Projektionsvorrichtung (23), die eingerichtet ist, einen Innenraum des Behälters (3) mit einem Satz an Bildern (11) zu bestrahlen, die von der elektromagnetischen Strahlung gebildet werden und die jeweils die Intraokularlinse (10) zeigen, und jedes der Bilder (11) des Satzes unter einem anderen Einstrahlwinkel (Θ) bezüglich einer durch den Innenraum verlaufenden Bezugsebene (20) in den Innenraum einzustrahlen, wobei die Herstellungsvorrichtung (1) eine Flüssigkeit (7) aufweist, die in dem Innenraum angeordnet ist und die mit der elektromagnetischen Strahlung aushärtbar ist, wobei in der Flüssigkeit (7) ein Aktuator (13), ein Solarmodul und/oder ein Sensor (18) angeordnet ist und die Herstellungsvorrichtung (1) eingerichtet ist, die Intraokularlinse (10) um den Aktuator (13), das Solarmodul und/oder den Sensor (18) herum zu bilden.

8. Herstellungsvorrichtung gemäß Anspruch 7, wobei die Herstellungsvorrichtung (1) eine Drehvorrichtung (21) aufweist, die eingerichtet ist, den Behälter (3) zusammen mit der Bezugsebene (20) in eine Rotation zu versetzen, deren Symmetrieachse (4) durch den Behälter (3) verläuft, und die Projektionsvorrichtung (23) einen Projektor (2) aufweist, der eingerichtet ist, die Bilder (11) des Satzes nacheinander in den Behälter (3) einzustrahlen.

9. Herstellungsvorrichtung gemäß Anspruch 8, wobei die Herstellungsvorrichtung (1) eine Mehrzahl der Behälter (3) und für jeden der Behälter (3) eine Drehvorrichtung (21) aufweist, die eingerichtet ist, den zugehörigen Behälter (3) in die Rotation zu versetzen, deren Symmetrieachse (4) durch den zugehörigen Behälter (3) verläuft, und die Projektionsvorrichtung (23) eingerichtet ist, in jeden Innenraum von jedem der Behälter (3) einen jeweiligen Satz an Bildern (11), die von der elektromagnetischen Strahlung gebildet werden und die jeweils eine Intraokularlinse (10) zeigen, einzustrahlen, wobei die Projektionsvorrichtung (23) eingerichtet ist, die Behälter (3) gleichzeitig zu bestrahlen.

## Claims

1. Method for producing an intraocular lens (10), including the steps of:
- providing a container (3) which is transparent to electromagnetic radiation and in which a liquid that is curable by the electromagnetic radiation is arranged;
- irradiating the liquid (7) with a set of images (11) formed by the electromagnetic radiation and each showing an intraocular lens (10), with each image (11) of the set being radiated into the liquid (7) at a different angle of incidence (Θ) with respect to a reference plane (20) that extends through the liquid, as a result of which the liquid (7) is cured and the cured liquid (7) forms the intraocular lens (10), with an actuator (13), a solar module and/or a sensor (18) being arranged in the liquid (7) and the intraocular lens (10) being formed around the actuator (13), the solar module and/or the sensor (18).

2. Method according to Claim 1, wherein the intraocular lens (10) is an accommodating intraocular lens (10) which is distinguished in that it comprises an optic body (15) which is particularly soft and consequently has the ability to deform, and thus displace its focal spot.

3. Method according to Claim 1 or 2, wherein the liquid (7) contains oxygen and/or a free-radical scavenger, with the oxygen and/or the free-radical scavenger being dissolved in the liquid (7).

4. Method according to any of Claims 1 to 3, wherein the images (11) are radiated into the liquid (7) simultaneously.

5. Method according to any of Claims 1 to 3, wherein the images (11) are successively radiated into the liquid (7) while the container (3) is rotated, with the reference plane (20) rotating together with the container (3).

6. Method according to any of Claims 1 to 5, including the step of:
- post-processing the intraocular lens (10) after the latter has been formed, turning and/or laser ablation in particular being used for said post-processing.

7. Production device for an intraocular lens (10), comprising a container (3) transparent to electromagnetic radiation and a projection device (23) configured to irradiate an interior of the container (3) with a set of images (11) formed by the electromagnetic radiation and each showing the intraocular lens (10), and configured to radiate each image (11) of the set into the interior at a different angle of incidence (Θ) with respect to a reference plane (20) that extends through the interior, the production device (1) containing a liquid (7) which is arranged in the interior and which is curable by the electromagnetic radiation, with an actuator (13), a solar module and/or a sensor (18) being arranged in the liquid (7) and the production device (1) being configured to form the intraocular lens (10) around the actuator (13), the solar module and/or the sensor (18).

8. Production device according to Claim 7, wherein the production device (1) comprises a turning device (21) configured to make the container (3) rotate together with the reference plane (20), the axis of symmetry (4) of said rotation running through the container (3), and the projection device (23) comprises a projector (2) configured to successively radiate the images (11) of the set into the container (3).

9. Production device according to Claim 8, wherein the production device (1) comprises a plurality of the containers (3) and, for each container (3), a turning device (21) configured to make the associated container (3) rotate, the axis of symmetry (4) of said rotation running through the associated container (3), and the projection device (23) is configured to radiate a respective set of images (11) formed by the electromagnetic radiation and each showing an intraocular lens (10) into the interior of every one of the containers (3), with the projection device (23) being configured to irradiate the containers (3) simultaneously.

## Revendications

1. Procédé de fabrication d'une lentille intraoculaire (10), comprenant les étapes de :
- mise à disposition d'un récipient (3) transparent au rayonnement électromagnétique, dans lequel est agencé un liquide qui est durcissable par le rayonnement électromagnétique ;
- irradiation du liquide (7) par un jeu d'images (11), qui sont formées par le rayonnement électromagnétique et qui représentent à chaque fois une lentille intraoculaire (10), chaque image (11) du jeu étant projetée dans le liquide sous un angle d'incidence (Θ) différent par rapport à un plan de référence (20) s'étendant à travers le liquide (7), suite à quoi le liquide (7) est durci et le liquide (7) durci forme la lentille intraoculaire (10), un actionneur (13), un module solaire et/ou un capteur (18) étant agencé(s) dans le liquide (7) et la lentille intraoculaire (10) étant formée autour de l'actionneur (13), du module solaire et/ou du capteur (18).

2. Procédé selon la revendication 1, la lentille intraoculaire (10) étant une lentille intraoculaire (10) d'accommodation, qui est **caractérisée en ce qu'**elle présente un corps optique (15) qui est particulièrement souple et présente donc l'aptitude à se déformer et donc à déplacer son point focal.

3. Procédé selon la revendication 1 ou 2, le liquide (7) présentant de l'oxygène et/ou un piège de radicaux, l'oxygène et/ou le piège de radicaux étant dissous dans le liquide (7).

4. Procédé selon l'une des revendications 1 à 3, les images (11) étant projetées simultanément dans le liquide (7).

5. Procédé selon l'une des revendications 1 à 3, les images (11) étant projetées dans le liquide (7) successivement et pendant que le récipient (3) tourne, le plan de référence (20) tournant conjointement avec la récipient (3).

6. Procédé selon l'une des revendications 1 à 5, présentant l'étape de :
- post-traitement de la lentille intraoculaire (10), après sa formation, en particulier un post-traitement par tournage et/ou abrasion au laser.

7. Dispositif de fabrication pour une lentille intraoculaire (10), présentant un récipient (3) transparent au rayonnement électromagnétique et un dispositif de projection (23), qui est conçu pour irradier un espace interne du récipient (3) par un jeu d'images (11) qui sont formées par le rayonnement électromagnétique et qui représentent à chaque fois la lentille intraoculaire (10) et chaque image (11) du jeu étant projetée dans l'espace interne sous un anglé d'incidence (Θ) différent par rapport à un plan de référence (20) s'étendant à travers l'espace interne, le dispositif de fabrication (1) présentant un liquide (7) qui est agencé dans l'espace interne et durcissable par le rayonnement électromagnétique, un actionneur (13), un module solaire et/ou un capteur (18) étant agencé(s) dans le liquide (7) et le dispositif de fabrication (1) étant conçu pour forme la lentille intraoculaire (10) autour de l'actionneur (13), du module solaire et/ou du capteur (18).

8. Dispositif de fabrication selon la revendication 7, le dispositif de fabrication (1) présentant un dispositif de rotation (21) qui est conçu pour mettre en rotation le récipient (3) conjointement avec le plan de référence (20), l'axe de symétrie (4) de ladite rotation s'étendant à travers le récipient (3) et le dispositif de projection (23) présentant un projecteur (2) conçu pour projeter successivement les images (11) du jeu dans le récipient (3).

9. Dispositif de fabrication selon la revendication 8, le dispositif de fabrication (1) présentant une pluralité de récipients (3) et, pour chaque récipient (3), un dispositif de rotation (21) qui est conçu pour mettre en rotation le récipient (3) associé, l'axe de rotation (4) de ladite rotation s'étendant à travers le récipient (3) associé et le dispositif de projection (23) étant conçu pour projeter dans chaque espace interne de chaque récipient (3) un jeu respectif d'images (11) qui sont formées par le rayonnement électromagnétique et qui représentent à chaque fois une lentille intraoculaire (10), le dispositif de projection (23) étant conçu pour irradier simultanément les récipients (3).
